Europäisches Patentamt

European Patent Office

Office européen des brevets

(11) Veröffentlichungsnummer: **0 416 138 A1**

# EUROPÄISCHE PATENTANMELDUNG

(21) Anmeldenummer: 89115857.8

(22) Anmeldetag: 28.08.89

(51) Int. Cl.⁵: **A61N 1/36**

(43) Veröffentlichungstag der Anmeldung:
**13.03.91 Patentblatt 91/11**

(84) Benannte Vertragsstaaten:
**DE FR GB IT NL SE**

(71) Anmelder: **Siemens Elema AB**
**Röntgenvägen 2**
**S-171 95 Solna 1(SE)**
(84) **SE**

(71) Anmelder: **Siemens Aktiengesellschaft**
**Wittelsbacherplatz 2**
**W-8000 München 2(DE)**
(84) **DE FR GB IT NL**

(72) Erfinder: **Engström, Jan, Dipl.-Ing.**
**Dagsverksvägen 213**
**S-163 55 Spanga(SE)**

(54) **Mit dem Körper eines Lebewesens zur Stimulation und/oder Überwachung einer physiologischen Funktion zusammenwirkendes medizinisches Gerät.**

(57) Die Erfindung betrifft ein mit dem Körper eines Lebewesens zur Stimulation und/oder Überwachung einer physiologischen Funktion zusammenwirkendes medizinisches Gerät (1), dessen Zusammenwirken mit dem Körper des Lebewesens durch eine Steuerelektronik (5,6) bestimmt ist, mittels derer ein das Zusammenwirken mit dem Körper des Lebewesens beeinflussender Parametersatz aus einem Speicher (7) abrufbar ist. Dabei ist vorgesehen, dass in dem Speicher (7) mehrere Parametersätze speicherbar sind, wobei mittels einer Schalteinrichtung (5,6,36,51,53) jeweils einer der gespeicherten Parametersätze zum Abruf freigebbar ist.

## MIT DEM KÖRPER EINES LEBEWESENS ZUR STIMULATION UND/ODER ÜBERWACHUNG EINER PHYSIO-LOGISCHEN FUNKTION ZUSAMMENWIRKENDES MEDIZINISCHES GERÄT

Die Erfindung betrifft ein mit dem Körper eines Lebewesens zur Stimulation und/oder Überwachung einer physiologischen Funktion zusammenwirkendes medizinisches Gerät, dessen Zusammenwirken mit dem Körper des Lebewesens durch eine Steuerelektronik bestimmt ist, mittels derer ein das Zusammenwirken mit dem Körper des Lebewesens beeinflussender Parametersatz aus einem Speicher abrufbar ist.

Bei derartigen Geräten kann es sich zum Beispiel um elektromedizinische Therapiegeräte zur elektrischen Stimulation von Muskelgewebe handeln.

Es sind Geräte der eingangs genannten Art bekannt, die als Herzschrittmacher ausgebildet sind. Sie weisen demzufolge Mittel zum Detektieren spontaner Herzschläge und Mittel zum Stimulieren der Herzmuskeltätigkeit auf, wobei die Steuererelektronik im Falle des Ausbleibens spontaner Herzschläge die Mittel zum Stimulieren zur Abgabe elektrischer Stimulationsimpulse aktiviert, und zwar derart, dass eine bestimmte, meist einstellbare Herzschlagfrequenz nicht unterschritten wird. Das Zusammenwirken des Herzschrittmachers mit dem Körper des Patienten ist durch Daten eines sogenannten Parametersatzes beeinflussbar, die in dem Herzschrittmacher speicherbar und mittels der Steuerelektronik abrufbar sind. Diese Daten betreffen z.B. die nicht zu unterschreitende Herzschlagfrequenz, die Empfindlichkeit der Mittel zum Detektieren und den Energiegehalt der mittels der Mittel zum Stimulieren abgegebenen Stimulationsimpulse. Dabei besteht die Möglichkeit, den Parametersatz insgesamt oder bezüglich einzelner Daten zu verändern. Bei implantierbaren Herzschrittmachern geschieht dies mittels eines externen Gerätes, eines sogenannten Programmers, auf telemetrischem und damit nichtinvasivem Wege. Dies wird als Programmierung bzw. Umprogrammierung bezeichnet. Normalerweise ist die Programmierung bzw. Umprogrammierung dem behandelnden Arzt vorbehalten. Da der Patient den behandelnden Arzt nur in grösseren Zeitabständen aufsucht, muss der Arzt die Daten des Parametersatzes so wählen, dass diese für die unterschiedlichsten Bedürfnisse des Patienten und die unteschiedlichsten Situationen eine ausreichende Wirksamkeit des Herzschrittmachers ermöglichen. Der programmierte Parametersatz stellt somit günstigstenfalls einen gelungenen Kompromiss dar. Unter diesen Umständen besteht der Wunsch, den Parametersatz auf einfache Weise den momentanen Bedürfnissen des Patienten anpassen zu können.

Der Erfindung liegt die Aufgabe zugrunde, ein Gerät der eingangs genannten Art so auszubilden, dass der das Zusammenwirken des Gerätes mit dem Körper des Lebewesens bestimmende Parametersatz auf einfache und umfassende Weise unterschiedlichen Bedürfnissen des Lebewesens angepasst werden kann, vorzugsweise ohne dass es eines Eingriffs des behandelnden Arztes bedarf.

Nach der Erfindung wird diese Aufgabe dadurch gelöst, dass in dem Speicher mehrere Parametersätze speicherbar sind, wobei mittels einer Schalteinrichtung jeweils einer der gespeicherten Parametersätze zum Abruf freigebbar ist. In dem erfindungsgemässen Gerät sind also mehrere Parametersätze gespeichert. Jedoch kann die Steuerelektronik jeweils nur auf denjenigen zugreifen, der mittels der Schalteinrichtung freigegeben ist, so dass bei geeigneter Ausbildung und Betätigung der Schalteinrichtung die Steuerelektronik jeweils den den momentanen Bedürfnissen angepassten Parametersatz abruft.

Gemäss einer bevorzugten Ausführungsform der Erfindung ist vorgesehen, dass die Schalteinrichtung eine Echtzeit-Uhr aufweist, mittels derer in Abhängigkeit von der Tageszeit einen für einen bestimmten Zeitraum vorgesehenen Parametersatz zum Abruf freigebbar ist. Es besteht also die Möglichkeit, z.B. für die Tageszeit, während der der Patient einer mit körperlicher Aktivität verbundenen Tätigkeit, z.B. seinem Beruf, nachgeht einen entsprechend angepassten Parametersatz zu speichern und für die Nachtzeit einen den Bedürfnissen des schlafenden Patienten angepassten Parametersatz zu speichern, wobei in Abhängigkeit von der Zeit der eine oder der andere Parametersatz zum Abruf freigegeben ist.

Eine Variante der Erfindung sieht vor, dass die Schalteinrichtung eine Bedieneinheit aufweist, mittels derer einer der Parametersätze zum Abruf freigebbar ist. Da nur die Wahl zwischen gespeicherten Parametersätzen, jedoch keine Veränderung der Daten der Parametersätze möglich ist, kann dieses Bediengerät durchaus dem Patienten selbst in die Hand gegeben werden. Dieser kann dann beispielsweise im Falle einer mit Bettlägrigkeit verbundenen Erkrankung einen Parametersatz wählen, der den Bedürfnissen eines bettlägrigen Patienten angepasst ist. Dabei ist für den Fall, dass das Gerät derart ausgebildet ist, dass es in den Körper des Lebewesens implantierbar ist, vorgesehen, dass die Bedieneinheit als externes Bediengerät ausgeführt ist, mittels dessen telemetrisch einer der Parametersätze zum Abruf freigebbar ist.

Eine weitere bevorzugte Ausführungsform der Erfindung sieht vor, dass die Schalteinrichtung in Abhängigkeit von der Intensität der physiologischen Funktion des Lebewesens einen der Parametersätze zum Abruf freigibt. Es besteht so beispielsweise die Möglichkeit, im Falle des Auftretens bestimmter an einem der jeweiligen physiologischen Funktion entsprechenden Signal erkennbarer anormaler Zustände, im Falle eines als Herzschrittmacher ausgebildeten Gerätes können z.B. anhand eines der Herztätigkeit entsprechenden Signales Tachykardien, Fibrillationen usw. erkannt werden, einen zur Beendigung dieses Zustands geeigneten oder diesem Zustand wenigstens angepassten Parametersatz freizugeben.

Eine Ausführungsform der Erfindung sieht vor, dass die Schalteinrichtung einen Parametersatz zum Abruf freigibt, indem sie ausschliesslich die zu dem jeweiligen Parametersatz gehörigen Speicherstellen des Speichers zur Adressierung freigibt. Da ohnehin irgendwelche Massnahmen zur Adressierung des Speichers getroffen sein müssen, ist so auf einfache Weise sichergestellt, dass die Steuerelektronik ausschliesslich auf den jeweils freigegebenen Parametersatz zugreifen kann.

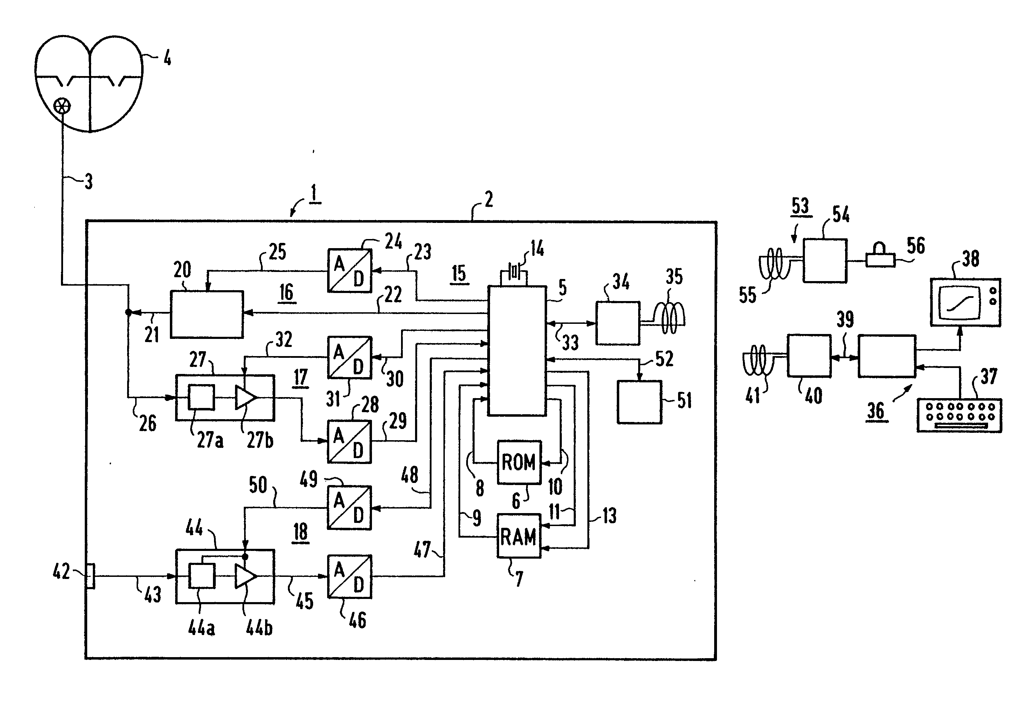

Ein Ausführungsbeispiel der Erfindung ist anhand der beigefügten einzigen Figur erläutert, die ein Blockschaltbild eines als Herzschrittmacher ausgebildeten erfindungsgemässen Gerätes zeigt.

In der Figur ist die Erfindung anhand eines insgesamt mit (1) bezeichneten Herzschrittmachers dargestellt. Die Bauteile des Herzschrittmachers (1) sind in einem schematisch angedeuteten hermetisch dichten Gehäuse (2) aufgenommen, das zur Implantation in den Körper eines Patienten geeignet ist. Von dem im VVI-Mode arbeitenden Herzschrittmacher (1) führt eine Elektrode (3) zu dem Herz (4) des Patienten und ist dort in ein Ventrikel, vorzugsweise das durch das Venensystem zugängliche rechte Ventrikel, eingepflanzt.

Der Herzschrittmacher (1) umfasst unter anderem einen Mikroprozessor (5), dem ein Nur-Lese-Speicher (ROM) (6) und ein Schreib-Lese-Speicher (RAM) (7) zugeordnet sind, die über Datenleitungen (8,9) und Adressleitungen (10,11) mit dem Mikroprozessor (5) in Verbindung stehen. Dabei weist die Adressleitung (11), die zu dem RAM (7) führt, eine Breite von 6 bit auf. Zu dem RAM (7) führt von dem Mikroprozessor (5) ausserdem eine Leitung (13), die zum Umschalten des RAM (7) von Schreib- auf Lesebetrieb und umgekehrt dient. In dem ROM (6) ist ein Programm gespeichert, mittels dessen sämtliche Funktionen des Herzschrittmachers (1) gesteuert werden. Wenn also im folgenden die Rede davon ist, dass der Mikroprozessor (5) eine bestimmte Funktion ausführt, so ist darunter zu verstehen, dass der Mikroprozessor (5) unter Ausführung des im ROM (6) gespeicherten Programmes und unter Heranziehung von im RAM (7) befindlichen Daten und ihm anderweitig, z.B.

über eine Eingangs-Beschaltung, zugeführter Daten zur Ausführung der jeweiligen Funktion tätig wird.

Mit dem Mikroprozessor (5) ist ein Quarz (14) verbunden, der zur Erzeugung der für den Betrieb des Mikroprozessors (5) erforderlichen Taktsignale dient und ausserdem die Zeitreferenz für den Betrieb des Herzschrittmachers (1) darstellt.

Der Mikroprozessor (5) des Herzschrittmachers (1) besitzt eine insgesamt mit (15) bezeichnete Eingangs/Ausgangs-Beschaltung, die mehrere Kanäle (16,17,18) aufweist.

Der Kanal (16) dient dazu, das Herz (4) erforderlichenfalls mit elektrischen Stimulationsimpulsen zu versorgen. Der Kanal (16) besitzt daher einen Stimulationsimpuls-Generator (20), dessen Ausgangsleitung (21) mit der Elektrode (3) verbunden ist. Der Stimulationsimpuls-Generator (20) ist über eine Leitung (22), die mit einem entsprechenden Ausgang des Mikroprozessors (5) verbunden ist, zur Abgabe eines elektrischen Stimulationsimpulses aktivierbar. Digitale Daten, die die Form der Stimulationsimpulse, z.B. deren Amplitude und Dauer, betreffen, gelangen von dem Mikroprozessor (5) über eine Leitung (23) zu einer Digital/Analog-Schnittstelle (24), die dem Stimulationsimpuls-Generator (20) über eine Steuerleitung (25) den digitalen Daten entsprechende analoge Steuersignale zuführt, die den Stimulationsimpuls-Generator (20) derart einstellen, dass er bei Bedarf Stimulationsimpulse eines gewünschten Energiegehaltes, also einer bestimmten Amplitude und bestimmten Zeitdauer, erzeugt.

Der Kanal (17) besitzt eine über eine Eingangsleitung (26) ebenfalls mit der Elektrode (3) verbundene Signalaufbereitungsschaltung (27), die dazu dient, ein mittels der Elektrode (3) vom Herzen (4) abgenommenes, der Aktivität des Herzens entsprechendes elektrisches Signal zu filtern und zu verstärken. Die Signalaufbereitungsschaltung (27) umfasst daher ein Filter (27a) und einen Verstärker (27b). Vom Ausgang der Signalaufbereitungsschaltung (27) gelangt das aufbereitete Signal zu einem Analog/Digital-Wandler (28). Von diesem gelangen über eine Leitung (29) digitale Daten zu einem entsprechenden Eingang des Mikroprozessors (5), die dem Verlauf des am Ausgang der Signalaufbereitungsschaltung (27) vorliegenden elektrischen Signales entsprechen, das seinerseits die elektrische Aktivität des Herzens (4) widerspiegelt. Der Mikroprozessor (5) ist über eine Leitung (30) mit einer Digital/Analog-Schnittstelle (31) verbunden, die ihr von dem Mikroprozessor (5) zugeführte digitale Daten als entsprechende analoge Signale über eine Steuerleitung (32) an die Signalaufbereitungsschaltung (27) weitergibt. Die digitalen Daten bzw. die entsprechenden analogen Signale dienen dazu, die Empfindlichkeit der Signalaufbereitungsschaltung (27) einzustellen, z.B. durch Änderung des Verstärkungsfaktors des Verstärkers (27b). Ausserdem besteht die Möglichkeit, den Verstärker (27b) vollständig zu sperren.

Die über die Leitung (29) dem Mikroprozessor (5) zugeführten digitalen Daten analysiert dieser daraufhin, ob in dem der Aktivität des Herzens (4) entsprechenden elektrischen Signal Ereignisse enthalten sind, die dem Auftreten eines natürlichen Herzschlages entsprechen. Detektiert der Mikroprozessor (5) einen natürlichen Herzschlag oder aktiviert er über die Leitung (22) den Stimulationsimpuls-Generator (20) zur Abgabe eines Stimulationsimpulses, beginnt der Mikroprozessor (5) als Zähler zu arbeiten und beginnt eine Anzahl von aus der Schwingung des Quarzes (14) abgeleiteten Taktimpulsen abzuzählen, die einem zwischen einer oberen und einer unteren Grenze einstellbaren Zeitintervall entspricht. Das jeweils eingestellte Zeitintervall bestimmt diejenige Stimulationsfrequenz, mit der das Herz (4) beim Ausbleiben natürlicher Herzschläge stimuliert wird. Gelangen während dieses Zeitintervalles über den Kanal (17) keine Daten zu dem Mikroprozessor (5), die dieser als natürlichen Herzschlag detektiert, aktiviert der Mikroprozessor (5) bei Ablauf des Zeitintervalls über die Leitung (22) den Stimulationsimpuls-Generator (20). Im Anschluss an die Abgabe eines Stimulationsimpulses beginnt der Mikroprozessor (5) erneut eine Anzahl von Taktimpulsen abzuzählen, die dem jeweils eingestellten, die Stimulationsfrequenz bestimmenden Zeitintervall ent spricht. Detektiert der Mikroprozessor (5) dagegen während des Laufs des jeweils eingestellten, die Stimulationsfrequenz bestimmenden Zeitintervalls einen natürlichen Herzschlag, bricht er den beschriebenen Zählvorgang ab, sofern ein weiteres Zeitintervall, die sogenannte Refraktärzeit, abgelaufen ist, und beginnt den beschriebenen Zählvorgang von neuem.

Die Refraktärzeit, die grundsätzlich kürzer ist als das zwischen beispielsweise 400 und 2000 ms einstellbare, die Stimulationsfrequenz bestimmende Zeitintervall, dauert zwischen etwa 250 und 450 ms (einstellbar). Die Refraktärzeit unterteilt sich in eine absolute Refraktärzeit mit einer festen Dauer von gewöhnlich 125 ms und eine relative Refraktärzeit, auf die der restliche Teil der gesamten jeweils eingestellten Refraktärzeit entfällt. Die Refraktärzeit beginnt jeweils gleichzeitig mit dem die Stimulationsfrequenz bestimmenden Zeitintervall zu laufen und wird von dem Mikroprozessor (5) im Zuge des gleichen Zählvorgangs ermittelt, der auch zur Ermittlung des die Stimulationsfrequenz bestimmenden Zeitintervalles dient. Während der absoluten Refraktärzeit ist im Kanal (17) der Verstärker (27b) der Signalaufbereitungsschaltung (27) vollständig gesperrt, was dadurch erreicht wird, dass der Mikroprozessor (5) den Verstärker (27b) über die Leitung (30), die Digital/ Analog-Schnittstelle (31) und die Steuerleitung (32) mit einem entsprechenden Steuersignal beaufschlagt. Infolge der vollständigen Sperrung des Verstärkers (27b) ist mittels des Mikroprozessors (5) während der Dauer der absoluten Refraktärzeit keinerlei Detektion möglich. Nach Ablauf der absoluten Refraktärzeit aktiviert der Mikroprozessor (5) den Verstärker (27b) wieder, so dass er in der Lage ist, natürliche Herzschläge zu detektieren. Detektiert der Mikroprozessor (5) während der relativen Refraktärzeit einen natürlichen Herzschlag, bricht er im Gegensatz zu einer Detektion nach Ablauf der Refraktärzeit den Zählvorgang zur Ermittlung des jeweils eingestellten, die Stimulationsfrequenz bestimmenden Zeitintervalles nicht ab, sondern führt ihn fort und schliesst ihn mit einer Aktivierung des Stimulationsimpuls-Generators (20) ab. Allerdings setzt der Mikroprozessor (5) nach der Detektion eines natürlichen Herzschlages nochmals die volle Refraktärzeit in Gang. Hierdurch wird erreicht, dass im Falle von Hochfrequenzstörungen, die zu Fehldetektionen führen, unabhängig vom Auftreten natürlicher Herzschläge Stimulationsimpulse mit der durch das jeweils eingestellte Zeitintervall bestimmten Stimulationsfrequenz erzeugt werden. Auch wenn die spontane Herzschlagfrequenz so hoch liegt, dass das Auftreten natürlicher Herzschläge jedesmal innerhalb der relativen Refraktärzeit erfolgt, erfolgt eine Abgabe von Stimulationsimpulsen mit der durch das jeweils eingestellte Zeitintervall bestimmten Stimulationsfrequenz, und zwar bis die spontane Herzschlagfrequenz unter eine Frequenz zurückgefallen ist, deren Periodendauer der jeweils eingestellten Refraktärzeit entspricht. Mittels dieser Funktion ist die Beendigung bestimmter Wiedereintrittstachykardien möglich.

Der Kanal (18) der Eingangs/Ausgangs-Schaltung (15) des Mikroprozessors (5) dient dazu, dem Mikroprozessor (5) Daten zur Verfügung zu stellen, die es diesem anhand des in dem ROM (6) gespeicherten Programmes gestatten, das der gewünschten Herzschlagfrequenz entsprechende Zeitintervall an die köperliche Aktivität des den Herzschrittmacher (1) tragenden Lebewesens anzupassen. Zu diesem Zweck ist ein piezoelektrischer Druck-Sensor (42) vorgesehen, der mit der Wandung des Gehäuses (2) verbunden ist. Während körperlicher Aktivitäten des Lebewesens entstehen durch die Bewegung der Muskeln und dergleichen mechanische Schwingungen im Körper des Lebewesens, die sich als Druckwellen in dem Körper des Lebewesens ausbreiten und von dem piezoelektrischen Sensor (42) aufgenommen und in elektrische Signale umgewandelt werden. Diese Signale, deren Amplitude mit zunehmender körperlicher Aktivität ebenfalls zunimmt, gelangen über eine Leitung (43) zu einer Signalaufbereitungsschaltung (44), die ein Filter (44a) mit nachfolgendem Verstärker (44b) enthält. Das Ausgangssignal der Signalaufbereitungsschaltung (44) gelangt über eine Leitung (45) zu einem Analog/Digital-Wandler (46) dessen digitale Ausgangssignale über eine Leitung (47) zu dem Mikroprozessor (5) gelangen. Der Mikroprozessor (5) ist über eine Leitung (48) mit einer Digital/Analog-Schnittstelle (49) verbunden, die ihr von dem Mikroprozessor (5) zugeführte digitale Daten als entsprechende analoge Signale über eine Steuerleitung (50) an die Signalaufbereitungsschaltung (44) weitergibt. Die digitalen Daten bzw. die diesen entsprechenden analogen Signale dienen dazu, die Empfindlichkeit der Signalaufbereitungsschaltung (44) einzustellen, z.B. durch Änderung des Verstärkungsfaktors des Verstärkers (44b). Ausserdem kann der Ausgang des Verstärkers (44b) vollständig gesperrt werden. In Abhängigkeit von dem zeitlichen Verlauf des von dem piezoelektrischen Sensor (42) stammenden Signals bzw. der entsprechenden digitalen Daten verändert der Mikroprozessor (5) in ähnlicher Weise, wie dies in der EP-A-0 080 348 beschrieben ist, das die Stimulationsfrequenz bestimmende Zeitintervall derart, dass dieses mit zunehmender körperlicher Aktivität verkürzt wird. Dies vollzieht sich zwischen einer unteren Grenze (Ruhepuls) und einer oberen Grenze (maximale Herzschlagfrequenz), die den Bedürfnissen des jeweiligen Lebewesens entsprechend gewählt werden. Entsprechende Daten werden telemetrisch in das RAM (7) eingegeben.

Der Mikroprozessor (5) ist über eine Leitung (33) mit einem Telemetrieschaltkreis (34) verbunden, an den eine Sende/Empfangs-Spule (35) angeschlossen ist. Der Herzschrittmacher (1) ist somit in der Lage, mit einem externen Programmiergerät (36) mit einer Tastatur (37) und einem Monitor (38) Daten auszutauschen, da das Programmiergerät (36) über eine Leitung (39) mit einem zweiten Telemetrieschaltkreis (40) verbunden ist, an den wieder eine Sende/Empfangs-Spule (41) angeschlossen ist. Zum Datenaustausch zwischen dem Herzschrittmacher (1) und dem Programmiergerät (36) wird die Sende/Empfangs-Spule (41) des zu dem Programmiergeräts (36) gehörigen Telemetrieschaltkreises (40) so auf der Körperoberfläche des den Herzschrittmacher (1) tragenden Lebewesens positioniert, dass sie mit der Sende-Empfangs-Spule (35) des Herzschrittmachers (1) induktiv gekoppelt ist. Es besteht dann die Möglichkeit, dem Programmiergerät (36) die in dem ROM (6) und dem RAM (7) befindlichen Daten zur Überprüfung bzw. Überprüfung und Veränderung zuzuführen. Ausserdem besteht die Möglichkeit, dem RAM (7) des Herzschrittmachers (1) von dem Programmiergerät (36) her veränderte bzw. zusätzliche Daten zuzuführen.

In dem RAM (7) sind u.a. Daten gespeichert, die

a) die Zeitdauer der Stimulationsimpulse,
b) die Amplitude der Stimulationsimpulse,
c) die Empfindlichkeit der Signalaufbereitungsschaltung (27) des Kanals (17),
d) die Empfindlichkeit der Signalaufbereitungsschaltung (44) des Kanals (18),
e) den oberen Grenzwert der Stimulationsfrequenz,
f) den unteren Grenzwert der Stimulationsfrequenz,
g) die Dauer der Refraktärzeit, und
h) eine einer verminderten körperlichen Aktivität entsprechende Herzschlagfrequenz festlegen.

Das RAM (7) besitzt eine Tiefe von 8 bit. Für jeden der Parameter a) bis h) kann also ein Datenwort mit einer Länge von 8 bit gespeichert werden.

Die unter a) bis h) angegebenen Parameter bilden einen das Zusammenwirken des Herzschrittmachers (1) mit dem Körper bzw. dem Herz (4) des Patienten beeinflussenden Parametersatz. Dabei sind in dem RAM (7) insgesamt sechs unterschiedliche Parametersätze gespeichert. Die Parameter a) bis h) sind im Falle jedes Parametersatzes unter den Adressen XXX000 bis XXX111 gespeichert. Die einzelnen Parametersätze sind unter den Adressen 000XXX bis 101XXX gespeichert. Dabei bedeutet die Angabe X, dass an dieser Stelle wahlweise die Ziffer 0 oder die Ziffer 1 stehen kann.

Unter der Adresse 011100 ist also der zu dem vierten Parametersatz gehörige obere Grenzwert der Stimulationsfrequenz gespeichert.

Welcher der Parametersätze abrufbar ist, d.h. von dem Mikroprozessor (5) über die Adressleitung (11) adressiert werden kann, so dass der Mikroprozessor (5) auf die Daten bezüglich der einzelnen Parameter a) bis h) zugreifen kann, hängt zunächst von Daten ab, die von einer Echtzeit-Uhr (51) über eine Leitung (52) zu dem Mikroprozessor (5) gelangen. Während der Zeit von 09.00 h bis 21.00 h adressiert der Mikroprozessor (5) den ersten Datensatz unter den Adressen 000XXX, der bezüglich der einzelnen Parameter a) bis h) Daten enthält, die den Bedürfnissen des Patienten während der genannten Zeit, in der dieser eine erhöhte körperliche Aktivität aufweist, entsprechen. In der Zeit von 23.00 h bis 06.00 h kann der Mikroprozessor (5) nur den zweiten Parametersatz unter den Adressen 001XXX adressieren, der den Bedürfnissen des schlafenden Patienten angepasst ist. In den verbleibenden Zeiträumen von 06.00 h bis 09.00 h und von 21.00 h bis 23.00 h ist nur der dritte Parametersatz unter den Adressen 010XXX zur Adressierung freigegeben. Der dritte Parametersatz trägt den Bedürfnissen des Patienten bei eingeschränkter körperlicher Aktivität Rechnung.

Der vierte Parametersatz der unter den Adressen 011XXX gespeichert ist, enthält bezüglich der Parameter a) bis h) Daten, die den Bedürfnissen des Patienten entsprechen, wenn dieser während des Tages, also in der Zeit von 06.00 h bis 23.00 h, keine körperliche Aktivität entwickelt, z.B. seinen Mittagsschlaf hält. Ist entweder der erste oder der dritte Parametersatz zur Adressierung freigegeben, vergleicht der Mikroprozessor (5) in kurzen Zeitabständen (Grössenordnung von Minuten) die Herzschlagfrequenz des Patienten bzw. im Falle der Stimulation die Stimulationsfrequenz mit dem einer eingeschränkten körperlichen Aktivität des Patienten entsprechenden Grenzwert der Herzschlagfrequenz gemäss h). Wird dieser Grenzwert für eine bestimmte Zeit, z.B. 15 Minuten, unterschritten, greift der Mikroprozessor (5) unabhängig davon, dass gemäss dem ihm von der Echtzeit-Uhr (51) zugeführten Daten entweder der erste oder dritte Parametersatz massgeblich ist, auf den vierten Parametersatz zu. Die anderen Parametersätze sind während dieser Zeit nicht zur Adressierung freigegeben. Sobald jedoch die Herzschlag- bzw. Stimulationsfrequenz den Grenzwert wieder übersteigt, wird der vierte Parametersatz gesperrt und der gemäss den von der Echtzeit-Uhr (51) zu dem Mikroprozessor (5) gelangenden Daten massgebliche Parametersatz zur Adressierung freigegeben.

Der fünfte Parametersatz, der unter den Adressen 100XXX erreichbar ist, ist den Bedürfnissen eines bettlägrig erkrankten Patienten angepasst. Dieser wird im Falle einer solchen Erkrankung mittels eines dem Patienten zur Verfügung stehenden Bediengerätes (53) telemetrisch zur Adressierung freigegeben. Das Bediengerät (53) umfasst einen Telemetrieschaltkreis (54), eine mit diesem verbundene Sendespule (55) und einen mit dem Telemetrieschaltkreis (54) verbundenen Taster (56). Wird der Taster (56) betätigt, gelangt ein Signal zu dem Herzschrittmacher (1), das den Mikroprozessor (5) veranlasst auf den fünften Parametersatz zuzugreifen. Die Parametersätze 1 bis 4 sind solange gesperrt, bis durch eine erneute Betätigung des Tasters (56) der fünfte Parametersatz wieder gesperrt wird, worauf der gemäss den vorstehend beschriebenen Kriterien massgebliche Parametersatz zur Adressierung freigegeben ist.

Der sechste Parametersatz, der unter den Adressen 101XXX erreichbar ist, enthält diagnostischen Zwecken angepasste Daten, die es dem behandelnden Arzt gestatten, das Zusammenwirken des Herzschrittmachers (1) mit dem Körper des Patienten zu beurteilen, wenn sich der Patient routinemässig oder wegen des Auftretens von Symptomen bei ihm vorstellt. Diesen Parametersatz kann der Arzt durch eine entsprechende Betätigung der Tastatur (37) des Programmiergerätes (36) freigeben. Der sechste Parametersatz ist dann für einen definierten Zeitraum, z.B. 10 Minuten, den der Mikroprozessor (5) anhand der ihm von der Echtzeit-Uhr (51) zugeführten Daten ermittelt, zur Adressierung freigegeben. Während dieses Zeitraumes sind alle übrigen Parametersätze gesperrt. Nach Ablauf des definierten Zeitraumes ist wieder derjenige Parametersatz zur Adressierung freigegeben, der gemäss den zuvor beschriebenen Kriterien massgeblich ist.

Mittels des Programmiergerätes (36) ist der behandelnde Arzt ausserdem in der Lage, zu diagnostischen Zwecken einen beliebigen der ersten fünf Parametersätze zur Adressierung freizugeben. Weiter ist der behandelnde Arzt in der Lage, mittels des Programmiergerätes (36) die bezüglich der Parameter a) bis h) in den Parametersätzen enthaltenen Daten zu verändern.

Unter den Adressen 110000 bis 111111 sind in dem RAM (7) Daten speicherbar, die keinem der Parametersätze zugeordnet sind und auf die der Mikroprozessor (5) ständig zugreifen kann. Bei diesen Daten, die mittels des Programmiergerätes (36) eingegeben werden können, kann es sich z.B. um Daten handeln, die zur Patientenidentifikation dienen.

Im Falle des erfindungsgemässen Herzschrittmachers (1) wird eine Vielzahl von Funktionen durch einen entsprechend programmierten Mikroprozessor (5) ausgeführt. Anstelle des Mikroprozessors (5) kann jedoch auch eine koventionell aufgebaute Steuerlogik vorgesehen sein.

Die Erfindung wurde ausschliesslich anhand eines Herzschrittmachers erläutert. Sie kann jedoch auch bei anderen Geräten zur Stimulation und/oder Überwachung einer physiologischen Funktion Verwendung finden.

Bezugszeichenliste

1 Herzschrittmacher
2 Gehäuse
3 Elektrode
4 Herz
5 Mikroprozessor
6 Nur-Lese-Speicher (ROM)
7 Schreib-Lese-Speicher (RAM)
8,9 Datenleitung
10,11 Adressleitung
13 Steuerleitung
14 Quarz
15 Eingangs/Ausgangs-Schaltung
16,17,18 Kanal
20 Stimulationsimpuls-Generator
21 Ausgangsleitung
22,23 Leitung
24 Digital/Analog-Schnittstelle
25 Steuerleitung
26 Eingangsleitung
27 Signalaufbereitungsschaltung

27a Filter

27b Verstärker

28 Analog/Digital-Wandler

29,30 Leitung

31 Digital/Analog-Schnittstelle

32 Steuerleitung

33 Leitung

34 Telemetrieschaltkreis

35 Sende/Empfangs-Spule

36 Programmiergerät

37 Tastatur

38 Monitor

39 Leitung

40 Telemetrieschaltkreis

41 Sende/Empfangs-Spule

42 Piezoelektrischer Drucksensor

43 Leitung

44 Signalaufbereitungsschaltung

44a Filter

44b Verstärker

45 Leitung

46 Analog/Digital-Wandler

47,48 Leitung

49 Digital/Analog-Schnittstelle

50 Steuerleitung

51 Echtzeit-Uhr

52 Leitung

53 Bediengerät

54 Telemetrieschaltkreis

55 Sendespule

56 Tastatur

**Ansprüche**

1. Mit dem Körper eines Lebewesens zur Stimulation und/oder Überwachung einer physiologischen Funktion zusammenwirkendes medizinisches Gerät, dessen Zusammenwirken mit dem Körper des Lebewesens durch eine Steuerelektronik (5,6) bestimmt ist, mittels derer ein das Zusammenwirken mit dem Körper des Lebewesens beeinflussender Parametersatz aus einem Speicher (7) abrufbar ist, **dadurch gekennzeichnet,** dass in dem Speicher (7) mehrere Parametersätze speicherbar sind, wobei mittels einer Schalteinrichtung (5,6,36,51,53) einer der gespeicherten Parametersätze zum Abruf freigebbar ist.

2. Gerät nach Anspruch 1, **dadurch gekennzeichnet,** dass die Schalteinrichtung (5,6,36,51, 53) eine Echtzeit-Uhr (51) aufweist, mittels derer in Abhängigkeit von der Tageszeit ein für einen bestimmten Zeitraum vorgesehener Parametersatz zum Abruf freigebbar ist.

3. Gerät nach Anspruch 1 oder 2, **dadurch gekennzeichnet,** dass die Schalteinrichtung eine Bedieneinheit (36,53) aufweist, mittels derer einer der Parametersätze zum Abruf freigebbar ist.

4. Gerät nach Anspruch 3, **dadurch gekennzeichnet,** dass das Gerät derart ausgebildet ist, dass es in den Körper des Lebewesens implantierbar ist, und dass die Bedieneinheit als externes Bediengerät (36,53) ausgeführt ist, mittels dessen telemetrisch einer der Parametersätze zum Abruf freigebbar ist.

5. Gerät nach Anspruch 3 oder 4, **dadurch gekennzeichnet,** dass ein mittels der Bedieneinheit (36,53) zum Abruf freigegebener Parametersatz mittels der Bedieneinheit (36,53) wieder sperrbar ist.

6. Gerät nach Anspruch 3 oder 4, **dadurch gekennzeichnet,** dass ein mittels der Bedieneinheit (36) zum Abruf freigegebener Parametersatz nach Ablauf eines definierten Zeitintervalls selbsttätig gesperrt wird.

7. Gerät nach einem der Ansprüche 1 bis 6, **dadurch gekennzeichnet,** dass die Schalteinrichtung (5,6) in Abhängigkeit von der Intensität der physiologischen Funktion einen der Parametersätze zum Abruf freigibt.

8. Gerät nach einem der Ansprüche 1 bis 7, **dadurch gekennzeichnet,** dass die Schalteinrichtung (5,6,36,51,53) einen Parametersatz zum Abruf freigibt, indem sie ausschliesslich die zu dem jeweiligen Parametersatz gehörigen Speicherstellen des Speichers (7) zur Adressierung freigibt.

9. Gerät nach einem der Ansprüche 1 bis 8, **dadurch gekennzeichnet,** dass Mittel (5,6,27) zum Detektieren von physiologischen Ereignissen und/oder Mittel (5,6,20) zum Stimulieren physiologischer Vorgänge vorgesehen sind.

10. Gerät nach Anspruch 9, **dadurch gekennzeichnet,** dass das Gerät als Herzschrittmacher (1) ausgebildet ist, wobei die Mittel (5,6,27) zum Detektieren die elektrische Aktivität des Herzens (4) überwachen und die Mittel (5,6,20) zum Stimulieren die Herztätigkeit mittels elektrischer Stimulationsimpulse stimulieren.

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X | EP-A-216725 (BIOTRONIK GMB)<br>* Zusammenfassung *<br>* Seite 15, Zeile 27 - Seite 19, Zeile 2 *<br>* Seite 20, Zeile 21 - Seite 28, Zeile 5 *<br>* Seite 30, Zeile 24 - Seite 31, Zeile 30 *<br>* Seite 33, Zeilen 1 - 32 *<br>* Seite 37, Zeilen 3 - 29 *<br>* Seite 40, Zeile 14 - Seite 42, Zeile 13;<br>Figuren 1-5 *<br>--- | 1, 3-5, 7-10 | A61N1/36 |
| X | EP-A-160801 (MEDTRONIC, INC)<br>* das ganze Dokument *<br>--- | 1, 3-10 | |
| X | EP-A-11936 (MEDTRONIC, INC)<br>* Zusammenfassung *<br>* Seite 6, Zeile 20 - Seite 28, Zeile 25 *<br>* Seite 66, Zeile 7 - Seite 68, Zeile 15;<br>Figuren *<br>--- | 1, 3-10 | |
| A | US-A-4562841 (B P.BROCKWAY ET AL.)<br>* das ganze Dokument *<br>--- | 1, 3-10 | **RECHERCHIERTE SACHGEBIETE (Int. Cl.5)** |
| A | WO-A-8607270 (G.GRASSI ET AL.)<br>* das ganze Dokument *<br>----- | 2 | A61N |

Der vorliegende Recherchenbericht wurde für alle Patentansprüche erstellt

| Recherchenort | Abschlußdatum der Recherche | Prüfer |
|---|---|---|
| DEN HAAG | 18 MAI 1990 | FERRIGNO A. |

KATEGORIE DER GENANNTEN DOKUMENTE

X : von besonderer Bedeutung allein betrachtet
Y : von besonderer Bedeutung in Verbindung mit einer
 anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
 nach dem Anmeldedatum veröffentlicht worden ist
D : in der Anmeldung angeführtes Dokument
L : aus andern Gründen angeführtes Dokument

................................................................
& : Mitglied der gleichen Patentfamilie, übereinstimmendes
 Dokument